# FASCICULE DE BREVET EUROPEEN

(11) **EP 4 237 048 B1**
(45) Date de publication et mention de la délivrance du brevet: **16.07.2025**
(21) Numéro de dépôt: 21801095.7
(22) Date de dépôt: 26.10.2021
(51) Int. Cl.: A61M 5/42, A61B 5/00, G06V 10/44, G06V 40/14, G06T 7/12, G06T 7/136

(54) **PROCÉDÉ ET SYSTÈME DE DÉTERMINATION D'UN SEGMENT D'INSERTION OPTIMALE DANS UN VAISSEAU SANGUIN D'UN PATIENT**
VERFAHREN UND SYSTEM ZUR BESTIMMUNG EINES OPTIMALEN EINSETZSEGMENTS IN EIN BLUTGEFÄSS EINES PATIENTEN
METHOD AND SYSTEM FOR DETERMINING AN OPTIMAL INSERTION SEGMENT IN A BLOOD VESSEL OF A PATIENT

(30) Priorité: 30.10.2020 FR 2011160
(43) Date de publication de la demande: 06.09.2023
(73) Titulaire: BHEALTHCARE, 44800 Saint-Herblain (FR)
(72) Inventeur: BRETEAU, Aliaume, 44000 Nantes (FR); DE CHAISEMARTIN, Jean-Baptiste, 44300 Nantes (FR); BACHELIER, Sophie, 44200 Nantes (FR)
(74) Mandataire: Bringer IP
(86) Numéro de dépôt international: PCT/EP2021/079632
(87) Numéro de publication internationale: WO 2022/090201

(56) Documents cités:
- US-A1- 2012 190 981
- SHAHZAD A ET AL: "Subcutaneous veins detection and backprojection method using Frangi vesselness filter", 2015 IEEE SYMPOSIUM ON COMPUTER APPLICATIONS & INDUSTRIAL ELECTRONICS (ISCAIE), IEEE, 12 April 2015 (2015-04-12), pages 65 - 68, XP032792873, DOI: 10.1109/ISCAIE.2015.7298329

## Description

### Domaine technique de l'invention

L'invention concerne un procédé et un système de détermination d'un segment d'insertion optimale dans un vaisseau sanguin d'un patient, humain ou non. L'invention concerne plus particulièrement un procédé et un système d'acquisition et de traitement d'images permettant la détermination d'un segment d'insertion optimale dans un vaisseau sanguin d'un patient. L'invention concerne également une machine de prélèvement sanguin automatique ou semi-automatique mettant en œuvre un système selon l'invention.

### Arrière-plan technologique

Les établissements de santé réalisent chaque jour un très grand nombre d'accès vasculaires, par exemple des prises de sang, des injections, etc. Ces opérations sont chronophages, répétitives et potentiellement dangereuses pour le personnel soignant et le patient compte tenu des risques de blessure qu'elles comportent liés par exemple à un tremblement du personnel soignant, sa fatigue, son inexpérience, un mauvais geste ou à un mauvais réflexe du patient lors de l'introduction de l'aiguille dans un vaisseau sanguin.

En outre, certains patients présentent des vaisseaux sanguins peu propices à une bonne prise de sang, ce qui peut nécessiter plusieurs tentatives d'insertion de l'aiguille par le personnel soignant avant de pouvoir atteindre un vaisseau sanguin permettant une prise de sang. Cette répétition des tentatives peut être douloureuse pour le patient et peut générer des blessures, ce qui ne fait que compliquer l'opération de prélèvement pour ces patients.

De plus, la crise sanitaire mondiale de coronavirus appelle le développement de système limitant les contacts entre les patients et le personnel soignant et/ou pouvant assurer des dépistages massifs de la population.

Ainsi, le déposant a proposé dans le document WO2015158978 une machine d'insertion automatique dans une veine d'un patient comprenant des moyens de capture d'image proche infrarouge du bras du patient, des moyens de détection d'une veine dans l'image capturée, un dispositif de maintien de la veine détectée, une aiguille et des moyens d'insertion de l'aiguille dans la veine détectée.

Un tel dispositif permet ainsi d'automatiser les opérations de prélèvement sanguin.

La détection de la veine par ce dispositif est une étape critique qui permet de s'assurer que l'insertion de l'aiguille se fait dans de bonnes conditions sanitaires et sécuritaires. En particulier, il existe un besoin de trouver un segment d'insertion optimale qui limite de façon importante tout risque, en permettant de s'assurer que la veine soit atteinte facilement. Le segment d'insertion se définit par un point d'insertion, une direction d'insertion et une longueur maximale d'insertion.

Des solutions ont été proposées dans l'art antérieur pour la détection des vaisseaux sanguin tels que les veines, en particulier pour assister un opérateur souhaitant effectuer une insertion manuelle en lui permettant de déterminer quelle veine semble la plus intéressante pour effectuer une insertion de l'aiguille. Ces solutions font intervenir des systèmes d'imagerie ou de détection de veine pouvant être coûteux, lents, et parfois difficiles à mettre en œuvre.

Toutefois, les solutions de l'art antérieur se limitent à une assistance à l'opérateur, l'opérateur déterminant lui-même le point d'insertion, et l'orientation de l'aiguille.

Les inventeurs ont ainsi cherché à améliorer la procédure de détermination d'un segment d'insertion optimale pour permettre notamment un fonctionnement automatique et autonome du choix du segment d'insertion optimale sur un grand nombre de patients de profils variés, c'est-à-dire quels que soient leur morphologie, leur pigmentation de peau, leurs marques, grains de beauté, poils, etc.

US 2012/190981 A1 décrit unsystème et un procédé d'insertion autonome d'aiguilles intraveineuses. Le système d'insertion intraveineuse autonome comprend un bras robotisé, un ou plusieurs capteurs fixés de manière pivotante au bras robotisé pour collecter des informations sur des sites d'insertion potentiels dans le bras d'un sujet, un dispositif médical fixé de manière pivotante au bras robotisé, et un contrôleur en communication avec les capteurs et le bras robotisé, le contrôleur recevant les informations des capteurs sur des sites d'insertion potentiels, et le contrôleur sélectionnant un site d'insertion cible et dirigeant le bras robotisé pour insérer le dispositif médical dans le site d'insertion cible.

HAHZAD A ET AL: "Subcutaneous veins detection and backprojection method using Frangi vesselness filter", 2015 IEEE SYMPOSIUM ON COMPUTER APPLICATIONS & INDUS TRIAL ELECTRONICS (ISCAIE), IEEE, 12 avril 2015 (2015-04-12), pages 65-68, DOI: 10.1109/ISCAIE.2015.7298329, décrit une méthode pour localiser les veines sous-cutanées à partir d'images NIR. La ligne centrale des veines est détectée à l'aide d'un filtrage de Frangi et rétroprojetée sur l'image NIR pour mettre en évidence les gros vaisseaux sanguins.

### Objectifs de l'invention

L'invention vise à fournir un système et un procédé de détermination d'un segment d'insertion optimale dans un vaisseau sanguin d'un patient.

L'invention vise en particulier à fournir un système et un procédé de détermination automatique et autonome d'un segment d'insertion optimale.

L'invention vise en particulier à fournir, dans au moins un mode de réalisation de l'invention, un système et un procédé de détermination fonctionnant sur un grand nombre de patients de profils variés, c'est-à-dire quels que soient leur morphologie, leur pigmentation de peau, leurs marques, grains de beauté, poils, etc.

L'invention vise en particulier à fournir, dans au moins un mode de réalisation de l'invention, un système et un procédé de détermination efficace, peu coûteux et facile à mettre en œuvre.

L'invention vise en particulier à fournir, dans au moins un mode de réalisation de l'invention, un système et un procédé de détermination permettant de connaître l'orientation du vaisseau sanguin et de connaître un chemin possible de l'aiguille lors de l'insertion de l'aiguille.

### Exposé de l'invention

Pour ce faire, l'invention concerne un procédé de détermination d'au moins un segment d'insertion optimale dans un vaisseau sanguin d'un patient pour l'insertion d'une aiguille dans ledit vaisseau sanguin, ledit segment étant représentatif d'un point d'insertion dans une partie du corps du patient, d'une direction d'insertion et d'une longueur maximale d'insertion, comprenant les étapes suivantes :
- une étape d'éclairage de la partie du corps du patient avec un éclairage proche infrarouge,
- une étape d'acquisition d'images proche infrarouge de la partie du corps du patient avec au moins une caméra,
- une étape de prétraitement des images acquises pour obtenir une image de vaisseaux sanguins visibles en surface de la partie du corps du patient, dite image prétraitée,
- une étape d'application d'un filtre de détection de structures linéaires à ladite image prétraitée pour obtenir une image, dite carte de profil vasculaire, qui identifie les vaisseaux sanguins visibles en surface de la partie du corps du patient,
- une étape de binarisation de la carte de profil vasculaire,
- une étape de squelettisation des vaisseaux sanguins sur la carte de profil vasculaire binarisée, configurée pour obtenir pour chaque vaisseau sanguin un squelette dudit vaisseau sanguin,
- une étape de définition de segments d'insertion à partir desdits squelettes des vaisseaux sanguins, pour chaque vaisseau sanguin,
- une étape de classement des segments d'insertion en fonction de paramètres de classement prédéterminés, de sorte à identifier un ou plusieurs segments d'insertion optimale.

Un procédé de détermination de segments d'insertion optimale selon l'invention permet ainsi de déterminer les segments d'insertion les plus favorables et d'établir un classement parmi ces segments d'insertion, pour garantir la réussite et la sécurité de l'insertion de l'aiguille dans le vaisseau sanguin du patient, en effectuant un traitement de l'image proche infrarouge permettant de caractériser au mieux les vaisseaux sous-cutanés. Les vaisseaux sanguins sous-cutanés sont par exemple des veines, des artères, des capillaires, selon l'application souhaitée lors de l'insertion de l'aiguille (prélèvement, injection, etc.).

Les différentes étapes du procédé de détermination permettent une détermination automatique et autonome du segment d'insertion optimale, de façon efficace, peu coûteuse, et simple à mettre en place. Contrairement aux solutions de l'art antérieur, l'invention ne se limite pas à une aide à un opérateur humain, par exemple en visualisant l'emplacement des vaisseaux sanguins, mais l'invention permet une définition précise des segments d'insertion, c'est-à-dire d'un point d'insertion où l'aiguille devra être insérée, une direction d'insertion c'est-à-dire l'axe selon lequel l'aiguille sera insérée, et une longueur maximale d'insertion, c'est-à-dire la longueur maximale de la partie de l'aiguille qui peut être insérée, l'aiguille pouvant atteindre la veine avant cette longueur maximale d'insertion selon l'orientation de l'aiguille et la profondeur de la veine. L'angle d'insertion d'une aiguille est généralement compris entre 15° et 30° par rapport à la surface de la peau, conformément aux pratiques médicales actuelles. La longueur maximale d'insertion correspond donc à la longueur d'aiguille pouvant être insérée permettant d'atteindre la veine à l'angle minimal d'insertion. Si l'aiguille est insérée à un angle supérieur, celle-ci atteindra la veine avec une longueur d'insertion inférieure à la longueur maximale d'insertion. Cette définition précise des segments d'insertion permet l'utilisation d'un matériel d'insertion automatique robotisé. En suivant ce segment, l'aiguille traversera les différentes couches constituantes de la peau (derme, épiderme, hypoderme, etc.) jusqu'à atteindre le vaisseau sanguin situé dans le tissu adipeux sous-cutané.

La détermination des segments d'insertion peut se faire en temps réel de sorte à déterminer en permanence le ou les segments d'insertion optimale en fonction de l'état des vaisseaux sanguins qui peut varier en fonction du temps. En particulier, le vaisseau sanguin peut se déformer ou rouler sous l'effet d'une force mécanique, par exemple lors de l'insertion de l'aiguille.

L'étape de prétraitement permet d'obtenir une image des vaisseaux sanguins sur la partie du corps du patient pour préparer au filtrage de l'étape suivante. En particulier, l'étape de prétraitement peut comprendre l'isolation et/ou l'élimination sur l'image d'aspérités de la peau tels que les poils, grains de beauté, tatouage, etc., ainsi qu'éventuellement les capillaires sanguins si l'on souhaite uniquement voir des veines ou des artères. L'objectif est de renforcer le contraste entre les vaisseaux sanguins sous-cutanés et la peau du patient.

Les contours du membre du patient peuvent être aussi détectés et éventuellement supprimés de l'image pour ne conserver que les données relatives aux emplacements des vaisseaux sanguins.

La carte de profil vasculaire est obtenue via les images proche infrarouges. En particulier, la différence d'absorption des rayons proche infrarouges entre les couches de la peau et l'hémoglobine (désoxygénée dans les veines, oxygénées dans les artères) contenue dans les vaisseaux sanguins sous-cutanés permet d'obtenir la carte de profil vasculaire sur laquelle ces vaisseaux sanguins sous-cutanés se distinguent du reste du patient (peau, muscle, os, etc.).

Le traitement effectué à la carte de profil vasculaire permet un fonctionnement sur un grand nombre de patients de profils variés, notamment les patients dont les veines sont difficiles à identifier en vision directe et/ou par le toucher par palpation de ladite veine, en particulier par un opérateur humain, par exemple biologiste ou infirmier. La méthode selon l'invention s'affranchit de la pigmentation de la peau et est ainsi applicable à tous les phototypes.

On entend par proche infrarouge une longueur d'onde comprise entre 0,7 et 3 µm. Cette définition correspond en particulier aux domaines infrarouges IR-A et IR-B tel que définis par la Commission Internationale de l'Éclairage (CIE). Pour la détection de vaisseaux sanguins dans une partie du corps d'un patient, l'intervalle des longueurs d'ondes préférentielles se situe entre 0,7 et 0,9 µm, en particulier pour la détection de veines dans un bras.

L'insertion de l'aiguille est fréquemment effectuée dans un membre du patient, généralement un bras du patient. L'insertion de l'aiguille se fait généralement dans la région de la fosse cubitale.

Le filtre de détection de structures linéaires s'apparente à un filtre de détection d'arêtes ou un filtre de détection de contour, permettant de détecter, sur une image, la présence de structures linéaires, en particulier les vaisseaux sanguins dans l'invention.

Le classement des segments d'insertion permet de sélectionner le meilleur segment candidat à l'insertion de l'aiguille.

Selon une variante de l'invention, une étape d'extraction de contours des vaisseaux sanguins identifiées dans la carte de profil vasculaire binarisée est effectuée préalablement à la squelettisation des vaisseaux sanguins.

Avantageusement et selon l'invention, les paramètres de classements prédéterminés pour le classement des segments d'insertion sont choisis parmi un ou plusieurs paramètres de la liste suivante :
- la localisation du segment par rapport à un schéma connu de positions de vaisseaux sanguins sur la partie du corps du patient ;
- l'intensité moyenne de l'ensemble des points du vaisseau sanguin compris à l'intérieur de contours du vaisseau sanguin correspondant au segment, calculée sur la carte de profil vasculaire ;
- la longueur du segment ;
- la profondeur du vaisseau sanguin au niveau du segment ;
- le diamètre du vaisseau sanguin au niveau du segment ;
- l'orientation du segment ;
- la présence ou l'absence d'aspérités de la peau sur le segment d'insertion ;
- une préférence du patient ;
- un historique d'insertion précédente au même patient.

Selon cet aspect de l'invention, le classement des segments d'insertion dépend d'un ou plusieurs paramètres, éventuellement pondérés, de sorte à effectuer une détermination optimisée du segment d'insertion optimale, notamment pour maximiser les chances de réussite de l'insertion de l'aiguille et limiter au maximum les risques de sécurité.

Les paramètres et les pondérations éventuelles des différents paramètres peuvent être choisis en fonction de plusieurs critères, par exemple l'indice de masse corporelle (IMC) du patient, son âge, le type d'aiguille utilisé, l'historique de prélèvement du patient, l'historique de prélèvement de la machine d'insertion, la préférence du personnel, les capacités mécaniques de la machine de prélèvement/injection, etc.

L'orientation du segment peut en particulier être lié aux capacité mécaniques de la machine effectuant l'insertion, car l'insertion de l'aiguille selon certains angles trop éloignés d'un axe nominal de travail d'une machine de prélèvement peut être impossible à effectuer (la machine d'insertion étant par exemple limitée à un intervalle entre -90° et 90° par rapport à un axe nominal de travail, ou un intervalle plus petit ou plus grand et pas forcément symétrique selon les machines d'insertion).

Les aspérités de la peau désignent notamment des grains de beauté, cicatrices, hématomes, pétéchies, tatouages, boutons, etc.

Avantageusement et selon l'invention, le filtre de détection de structures linéaires est un filtre de Frangi.

Selon cet aspect de l'invention, le filtre de Frangi est particulièrement adapté pour la détection de vaisseaux sanguins. Il permet d'obtenir une détection précise des vaisseaux sanguins qui permettent, dans les étapes suivantes, d'obtenir une squelettisation précise des vaisseaux sanguins ce qui optimise la détermination de segments d'insertion. Le filtre de Frangi est en outre indépendant de l'échelle utilisée (filtre multi-échelles), ce qui garantit une détection efficace d'un patient à l'autre, quelles que soient les possibilités de différences de profil des vaisseaux sanguins sur l'image prétraitée.

Selon d'autres variantes de l'invention, d'autres types de filtres ou des combinaisons de filtres peuvent être utilisés, en particulier des filtres de dérivée seconde, etc.

Avantageusement et selon l'invention, l'étape de définition de segments d'insertion à partir des squelettes des vaisseaux sanguins comprend :
- une sous-étape de création d'un nœud pour chaque point de chaque squelette ;
- une sous-étape de caractérisation de chaque nœud pour former un graphe, un nœud étant un point terminal s'il n'est relié qu'à un seul nœud, une branche étant formée d'un ensemble de nœuds reliés entre eux n'ayant que deux nœuds voisins, chaque branche étant pondérée par le nombre de nœuds qui la forme ;
- une sous-étape de vérification de chaque graphe par comparaison de chaque branche avec le vaisseau sanguin correspondant sur l'image binarisée ;
- une sous-étape de correction de chaque branche non-centrée sur le vaisseau sanguin correspondant par division de la branche en nouvelles branches et création de nœuds de jonction entre les chaque nouvelles branches ;
- une sous-étape de définition des segments, un segment correspondant à une branche centrée sur son vaisseau sanguin correspondant et de longueur supérieure à un paramètre prédéterminé.

Selon cet aspect de l'invention, la définition des segments d'insertion s'effectue par approximation des squelettes par un graphe et par utilisation des branches de ce graphe pour former les segments. Par les sous-étapes de vérification et de correction, qui peuvent être exécutées autant de fois que nécessaire, on s'assure que chaque branche est bien centrée sur l'image binarisée du vaisseau sanguin correspondant, c'est-à-dire qu'elle est bien au centre du vaisseau sanguin, en particulier au centre des contours extraits du vaisseau sanguin, et d'une longueur suffisamment importante pour permettre une insertion de l'aiguille.

Ces sous-étapes permettent de faire un premier tri et de faire une liste des segments qui pourraient être utilisés pour l'insertion de l'aiguille, selon leur classement à la suite du procédé de détermination.

Les graphes peuvent comprendre un ou plusieurs nœuds reliés à au moins trois branches, qui forment des nœuds de jonction. Ils témoignent de la topologie de l'objet binaire correspondant au vaisseau sanguin puisqu'ils permettent de garder une marque d'une branche secondaire liée à une irrégularité ou variation de la forme de l'objet.

De préférence, avant la sous-étape de vérification, l'étape de définition de segments d'insertion à partir des squelettes des vaisseaux sanguins comprend une sous-étape de simplification du graphe pour obtenir un graphe final, par suppression de chemins les plus courts entre chaque point terminal, un chemin le plus long conservé étant le chemin comprenant les branches les plus pondérées. C'est ce graphe final qui est utilisé dans les sous-étapes suivantes. La recherche des segments les plus courts peut être effectuée grâce à un algorithme de recherche du plus court chemin, de préférence un algorithme de Dijkstra.

De préférence, la sous-étape de correction de chaque branche non-centrée comprend :
- une vérification d'un critère de centrage de la branche sur le vaisseau sanguin ;
- une recherche de points critiques d'une branche empêchant le respect du critère de centrage ;
- une division de la branche point par point à partir du point critique, pour former les nouvelles branches, et la division de nouvelles branches si nécessaire ;
- une nouvelle sous-étape de correction de chaque nouvelle branche non-centrée.

De préférence, la sous-étape de définition des segments comprend une suppression de segments doublons, si deux segments associés au même vaisseau sanguin sont superposés à plus de 65%.

Avantageusement et selon l'invention, la caméra est monochromatique et équipée d'un filtre passe-haut proche infrarouge.

Selon cet aspect de l'invention, les images acquises par la caméra sont directement filtrées par le filtre passe-haut et l'obtention de la carte de profil vasculaire est facilement obtenue via le prétraitement.

On entend par caméra tout dispositif permettant d'acquérir des images, et permettant d'enregistrer et/ou de transmettre les données de ces images pour traitement. Une caméra peut être composée d'un boitier comprenant un capteur d'acquisition d'image et un objectif. Le filtre passe-haut peut être agencé sur le capteur ou sur l'objectif selon les modes de réalisation. La caméra est configurée pour capter au minimum les longueurs d'ondes correspondant aux longueurs d'ondes souhaitées pour la définition des segments d'insertion, mais peut être configurée pour capter un intervalle de longueur d'onde plus large, auquel cas le filtre passe-haut permet de cibler les longueurs d'ondes souhaitées.

Selon d'autres variantes de l'invention, le filtrage proche infrarouge est effectué numériquement sur les images obtenues par la caméra, la caméra peut être configurée pour acquérir plusieurs couleurs (caméra RGB), etc.

Le procédé peut utiliser des images provenant de plusieurs caméras, dont les images sont alignées entre-elles.

L'invention concerne également un système de détermination d'au moins un segment d'insertion optimale dans un vaisseau sanguin d'un patient pour l'insertion d'une aiguille dans ledit vaisseau, ledit segment étant représentatif d'un point d'insertion dans une partie du corps du patient, d'une direction d'insertion et d'une longueur maximale d'insertion, comprenant une unité d'acquisition d'images de la partie du corps du patient et une unité de traitement des images acquises par ladite unité d'acquisition d'images, caractérisé en ce que ladite unité d'acquisition d'images comprend :
- un éclairage proche infrarouge configuré pour éclairer la partie du corps du patient avec un éclairage proche infrarouge, et
- au moins une caméra configurée pour acquérir des images proche infrarouges de la partie du corps du patient,
et en ce que l'unité de traitement d'image comprend :
- un module de prétraitement d'images configuré pour pouvoir fournir une image des vaisseaux sanguins visibles en surface de la partie du corps du patient, dite image prétraitée,
- un module de filtrage, configuré pour appliquer un filtre de détection de structures linéaires à ladite image prétraitée pour obtenir une image, dite carte de profil vasculaire, qui identifie les vaisseaux sanguins visibles en surface de la partie du corps du patient,
- un module de binarisation de la carte de profil vasculaire,
- un module de squelettisation des vaisseaux sanguins sur la carte de profil vasculaire binarisée, configuré pour obtenir pour chaque vaisseau sanguin un squelette dudit vaisseau sanguin,
- un module de définition de segments d'insertion à partir desdits squelettes des vaisseau sanguins, pour chaque vaisseau sanguin, et
- un module de classement des segments d'insertion en fonction de paramètres de classement prédéterminés, configuré pour identifier un ou plusieurs segments d'insertion optimale.

Avantageusement, le système de détermination selon l'invention met en œuvre le procédé de détermination selon l'invention.

Avantageusement, le procédé de détermination selon l'invention est mis en œuvre par le système de détermination selon l'invention.

L'invention concerne également une machine d'insertion automatique ou semi-automatique, pour l'insertion d'une aiguille dans une partie du corps d'un patient, par exemple un membre du patient, de préférence un bras du patient, comprenant un ensemble mécatronique tel qu'un bras robotisé, une unité de commande dudit ensemble mécatronique, et une tête d'insertion d'une aiguille montée sur l'ensemble mécatronique, caractérisé en ce qu'elle comprend en outre, un système de détermination selon l'invention configuré pour déterminer un segment d'insertion optimale de l'aiguille dans la partie du corps du patient.

Une machine d'insertion automatique ou semi-automatique équipée d'un système de détermination selon l'invention peut, de façon automatique ou semi-automatique et autonome, réaliser l'insertion de l'aiguille sur la partie du corps du patient (pour un prélèvement de sang ou injection), en se basant sur un des segments d'insertion optimale déterminé par le système de détermination. L'aiguille peut être reliée à une seringue, à un cathéter, etc.

La machine d'insertion peut être une machine de ponction ou de prélèvement si elle est destinée à prélever du sang, ou une machine d'injection si elle est destinée à injecter un produit dans le vaisseau sanguin.

L'invention concerne également un procédé de détermination, un système de détermination et une machine d'insertion caractérisés en combinaison par tout ou partie des caractéristiques mentionnées ci-dessus ou ci-après.

### Liste des figures

D'autres buts, caractéristiques et avantages de l'invention apparaîtront à la lecture de la description suivante donnée à titre uniquement non limitatif et qui se réfère aux figures annexées dans lesquelles :
[Fig. 1] est une vue schématique d'un procédé de détermination selon un mode de réalisation de l'invention,
[Fig. 2] est une représentation d'une image photographique prétraitée obtenue lors de la mise en œuvre d'un procédé de détermination selon un mode de réalisation de l'invention,
[Fig. 3] est une carte de profil vasculaire obtenu lors de la mise en œuvre d'un procédé de détermination selon un mode de réalisation de l'invention,
[Fig. 4] est une image binarisée obtenue lors de la mise en œuvre d'un procédé de détermination selon un mode de réalisation de l'invention,
[Fig. 5] est une image représentant la squelettisation des vaisseau sanguins obtenue lors de la mise en œuvre d'un procédé de détermination selon un mode de réalisation de l'invention,
[Fig. 6] est une image représentant des graphes représentatifs des squelettes des vaisseaux sanguins obtenues lors de la mise en œuvre d'un procédé de détermination selon un mode de réalisation de l'invention,
[Fig. 7] est une image représentant des segments issus des graphes obtenues lors de la mise en œuvre d'un procédé de détermination selon un mode de réalisation de l'invention,
[Fig. 8] est une image représentant les segments déterminés, rapportés sur l'image prétraitée obtenue lors de la mise en œuvre d'un procédé de détermination selon un mode de réalisation de l'invention,
[Fig. 9] est une vue schématique d'un système de détermination selon un mode de réalisation de l'invention,
[Fig. 10] est une vue schématique d'une machine d'insertion selon un mode de réalisation de l'invention.

### Description détaillée d'un mode de réalisation de l'invention

Sur les figures, les échelles et les proportions ne sont pas strictement respectées et ce, à des fins d'illustration et de clarté.

En outre, les éléments identiques, similaires ou analogues sont désignés par les mêmes références dans toutes les figures.

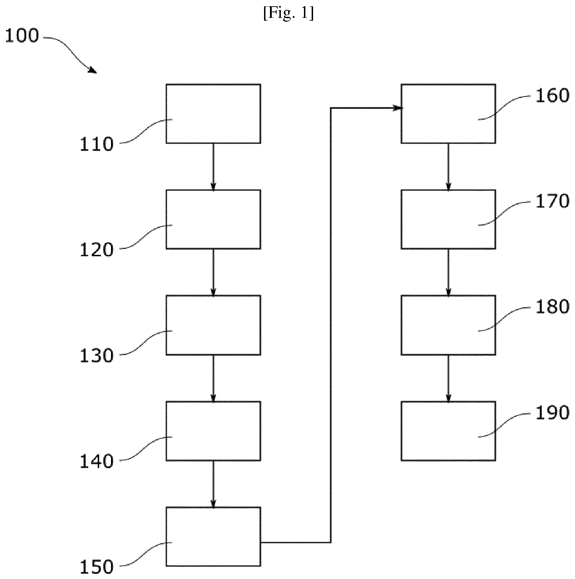

La figure 1 est une vue schématique d'un procédé de détermination selon un mode de réalisation de l'invention. Le procédé permet la détermination d'au moins un segment d'insertion optimale dans une partie du corps d'un patient pour l'insertion d'une aiguille dans un vaisseau sanguin du patient, ledit segment étant représentatif d'un point d'insertion dans ladite partie du corps du patient, d'une direction d'insertion et d'une longueur maximale d'insertion. Le vaisseau sanguin est par exemple une veine, une artère ou un capillaire.

Le procédé comprend une étape 110 d'éclairage de la partie du corps du patient, par exemple un membre du patient, comme ici un bras du patient, avec un éclairage proche infrarouge. L'éclairage proche infrarouge est composé d'une ou plusieurs lumières proche infrarouges. Une pluralité de lumières proche infrarouges permet d'éclairer de façon homogène la partie du corps du patient du patient qui présente une surface volumique qui peut créer des zones d'ombres si le nombre de lumières est insuffisant. L'objectif est d'éclairer la zone d'intérêt uniformément.

Le procédé comprend ensuite une étape 120 d'acquisition d'images proche infrarouge de la partie du corps du patient avec au moins une caméra. L'utilisation de l'éclairage proche infrarouge associé à l'acquisition par la caméra permet d'obtenir des images de réflectance proche infrarouge, appelée spectroscopie proche infrarouge.

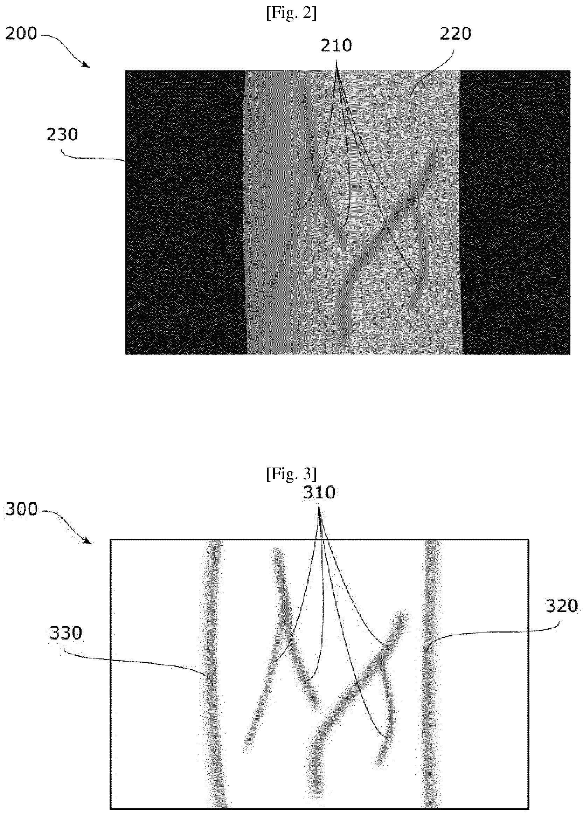

Le procédé comprend ensuite une étape 130 de prétraitement des images acquises pour obtenir une image des vaisseaux sanguins visibles en surface de la partie du corps du patient, dite image prétraitée. Une représentation d'une image prétraitée obtenue par ladite étape 130 de prétraitement est par exemple représentée en référence avec la figure 2. La figure 2 correspond à une représentation d'une image photographique telle que capturée par une caméra monochrome, puis prétraitée. La peau du membre 220 du patient présente des nuances de gris d'intensité variable selon le phototype du patient et l'absorption des rayons proche infrarouges de la peau. La différence d'absorption des rayons proche infrarouges entre les couches de la peau et l'hémoglobine contenue dans les vaisseaux sanguins sous-cutanés permet de distinguer les vaisseaux sanguins 210 du membre 220 du patient sur l'image 200 prétraitée. Un fond 230 sombre sur l'image 200 prétraitée, permet de bien délimiter les limites du membre 220.

L'étape de prétraitement comprend également des traitements permettant d'obtenir une image prétraitée optimisée, par exemple aucun, un ou plusieurs des traitements suivants :
- un seuillage ou un algorithme des k-moyennes pour réduire la zone à traiter par une binarisation de l'image ;
- une égalisation d'histogramme pour accentuer le contraste entre les vaisseaux et la peau ;
- application d'un filtre pour éliminer les informations susceptibles de gêner à l'exécution des étapes suivantes, par exemple un regroupement de poils sur la partie du corps du patient. Le filtre appliqué est par exemple un filtre médian, un filtre gaussien ou un filtre bilatéral.

Le procédé comprend ensuite une étape 140 d'application d'un filtre de détection de structures linéaires à ladite image prétraitée pour obtenir une image, dite carte de profil vasculaire, qui identifie les vaisseaux visibles en surface de la partie du corps du patient. Une carte de profil vasculaire obtenue par ladite étape 140 d'application du filtre est par exemple représentée en référence avec la figure 3. Le filtre de détection de structures linéaires appliqué ici est un filtre de Frangi. L'image 300 filtrée par ledit filtre de Frangi permet de conserver dans l'image uniquement les structures linéaires, en particulier les vaisseaux 310 sanguins et les contours 320, 330 du membre du patient.

Le procédé comprend ensuite une étape 150 de binarisation de la carte de profil vasculaire. Cette étape consiste à obtenir une image comprenant uniquement deux valeurs de pixel, par seuillage des luminosités.

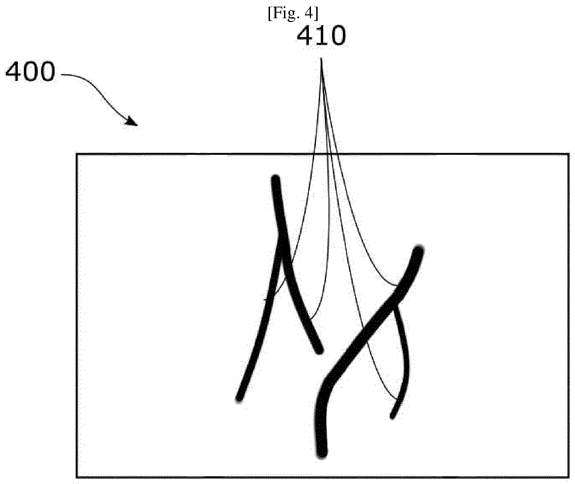

Le procédé comprend ensuite, dans ce mode de réalisation une étape 160 d'extraction de contours des vaisseaux sanguins identifiées dans la carte de profil vasculaire binarisée ; une carte 400 de profil vasculaire binarisée avec les vaisseaux sanguins obtenues par lesdites étape 150 de binarisation et étape 160 d'extraction de contours est par exemple représentée en référence avec la figure 4. Les contours du membre du patient peuvent être supprimés de l'image binarisée pour ne conserver que les données relatives aux emplacements des vaisseaux 410 sanguins.

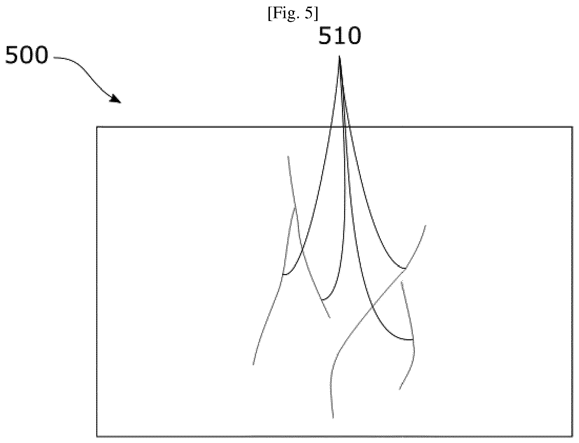

Le procédé comprend ensuite une étape 170 de squelettisation des vaisseaux sanguins, par exemple à partir des contours extraits, ou directement à partir de la carte de profil vasculaire binarisée, configurée pour obtenir pour chaque vaisseau sanguin un squelette dudit vaisseau sanguin. Les squelettes 510 des vaisseaux sanguins obtenus par ladite étape 170 sont par exemple représentés sur une image 500 binaire squelettisée en référence avec la figure 5. La squelettisation est par exemple réalisée par un algorithme de squelettisation, par exemple une squelettisation morphologique ou une squelettisation de Zhang-Suen. Le résultat de cette squelettisation permet d'obtenir les squelettes 510, un ensemble de courbes décrivant chacune le centre de l'objet vasculaire qui a été affiné sans détérioration de sa topologie.

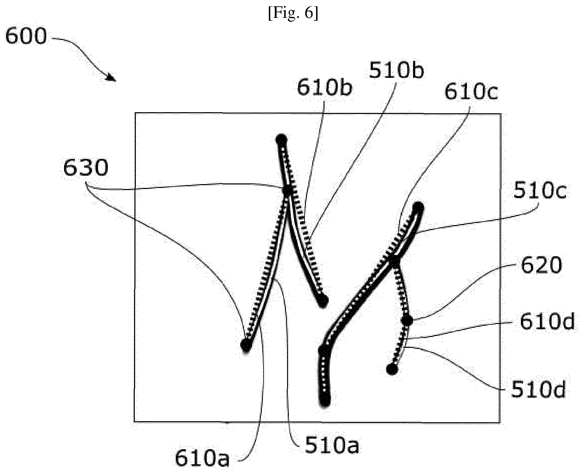

Le procédé comprend ensuite une étape 180 de définition de segments d'insertion à partir desdits squelettes des vaisseaux sanguins, pour chaque vaisseau sanguin. La figure 6 représente schématiquement des graphes 610a, 610b, 610c, 610d représentatifs des squelettes des vaisseaux sanguins, en correspondance avec l'image 600 filtrée binarisée pour faire apparaitre la correspondance de chaque graphe avec les contours des vaisseaux sanguins et les squelettes. Les graphes 610a, 610b, 610c, 610d, en lignes pointillées, représentent des approximations des squelettes 510a, 510b, 510c, 510d, représentés en lignes blanches. Un nœud est tout d'abord créé pour chaque point du squelette. Chaque nœud est ensuite caractérisé pour former un graphe, un nœud étant un point terminal s'il n'est relié qu'à un seul nœud, une branche étant formée d'un ensemble de nœuds reliés entre eux n'ayant que deux nœuds voisins, chaque branche étant pondérée par le nombre de nœuds qui la forme. Des nœuds de jonction sont reliés à au moins trois branches. Par exemple, le graphe 610a est une approximation du squelette 510a, et comprend notamment des points 630 terminaux. Le graphe 610d est une approximation du squelette 510d, et comprend notamment un nœud 620 de jonction.

Une branche entre deux nœuds d'un graphe représente une portion du squelette sensiblement rectiligne. Toutefois, une branche est une approximation du squelette et peut parfois être décentré du vaisseau sanguin que le squelette représente, comme visible par exemple pour les graphes 610b et 610d de la figure 6.

Le graphe peut être simplifié pour obtenir un graphe final, par suppression de chemins les plus courts entre chaque point terminal, un chemin le plus long conservé étant le chemin comprenant les branches les plus pondérées. C'est ce graphe final qui est utilisé dans les sous-étapes suivantes. La recherche des segments les plus courts peut être effectuée grâce à un algorithme de recherche du plus court chemin, de préférence un algorithme de Dijkstra.

Un traitement des graphes consiste alors à vérifier, dans une sous-étape de vérification de chaque graphe par comparaison avec le vaisseau sanguin correspondant sur l'image binarisée, puis corriger chaque branche non-centrée sur le vaisseau sanguin correspondant par division de la branche en deux nouvelles branches et création d'un nœud de jonction entre les deux nouvelles branches.

En particulier, la correction de chaque branche non-centrée comprend :
- une vérification d'un critère de centrage de la branche sur le vaisseau sanguin ;
- une recherche de points critiques d'une branche empêchant le respect du critère de centrage ;
- une division de la branche point par point à partir du point critique, pour former les nouvelles branches, et la division de nouvelles branches si nécessaire ;
- une nouvelle sous-étape de correction de chaque nouvelle branche non-centrée.

Une fois supprimé les branches de longueur inférieure à un paramètre prédéterminé et une fois toutes les branches centrées, on obtient des segments d'insertion qui correspondent auxdites branches conformes. Si on obtient des segments doublons, c'est à dire si deux segments associés au même vaisseau sanguin sont superposés à plus de 65%, on supprime ces segments.

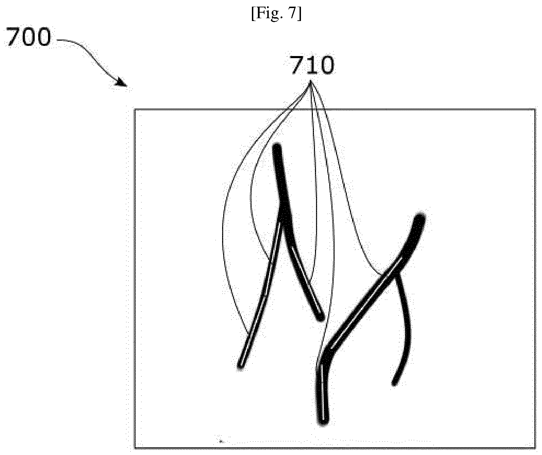

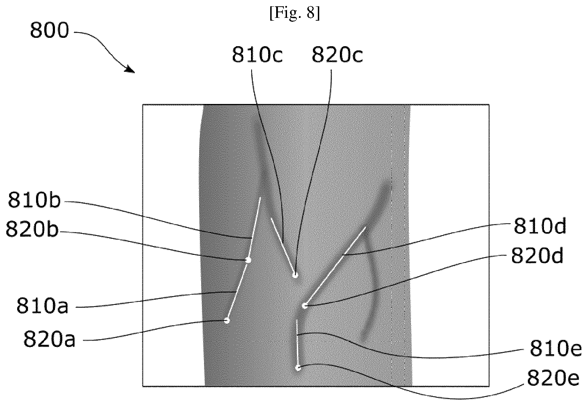

Ces segments 710 sont visibles en référence avec la figure 7 représentant les segments sur une image 700 binarisée des vaisseaux sanguins. Si on ramène les segments d'insertion à l'image prétraitée, on obtient une image 800 représentant les segments déterminés, rapportés sur l'image prétraitée telle que représentée à la figure 8. Les segments 810a, 810b, 810c, 810d d'insertion sont caractérisés respectivement par leur point 820a, 820b, 820c, 820d d'insertion, leur direction d'insertion et leur longueur maximale d'insertion. Le point d'insertion est calculé comme le point le plus prometteur parmi les deux extrémités. Le point le plus prometteur est par exemple calculé en fonction de l'orientation de la partie du corps du patient, des possibilités de la machine d'insertion, etc. En particulier, pour l'insertion dans le bras d'un patient, le point d'insertion est généralement le plus proche de la fosse antécubitale.

Le procédé comprend enfin une étape 190 de classement des segments d'insertion en fonction de paramètres de classement prédéterminés, de sorte à identifier un ou plusieurs segments d'insertion optimale.

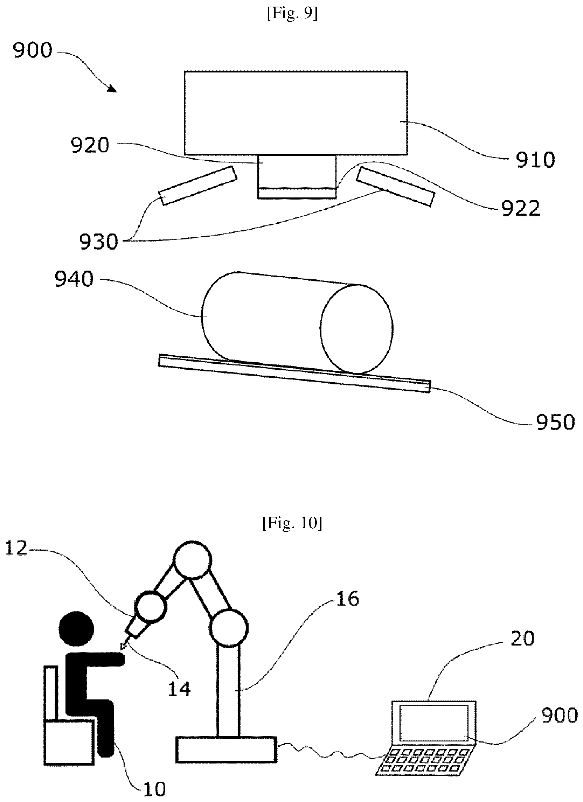

La figure 9 est une vue schématique d'un système de détermination selon un mode de réalisation de l'invention. Le système 900 de détermination comprend une unité 910 de traitement d'images comprenant un ensemble de modules configurés pour mettre en œuvre le procédé de détermination décrit précédemment. Un module décrit une brique matérielle et/ou logicielle permettant d'exécuter une ou plusieurs des étapes du procédé décrites précédemment. Plusieurs modules peuvent être compris dans un seul composant électronique et/ou un seul logiciel, ou l'étape mise en œuvre par un module peut nécessiter plusieurs composants électroniques et/ou logiciels. Les différents composants électroniques peuvent être assemblés sur une carte électronique.

L'unité 910 de traitement reçoit des images acquises par une unité d'acquisition d'image comprenant notamment une caméra 920 proche infrarouge et un éclairage 930 proche infra-rouge composé par exemple ici d'une pluralité de diodes électroluminescentes (plus communément appelées LED pour *Light Emitting Diode* en anglais) autour de la caméra 920. La caméra 920 et l'éclairage 930 peuvent être pilotées par un module de pilotage (non représenté), par exemple agencé sur une même carte électronique qu'un ou plusieurs modules de l'unité 910 de traitement d'image. La caméra 920 peut être équipée d'un filtre 922 proche infrarouge. La caméra 920 est généralement composée d'un boitier, comprenant un capteur, et d'un objectif comprenant des lentilles permettant la configuration de la focale et l'ouverture souhaitée (non représentés). Le filtre 922 proche infrarouge peut être agencé sur l'objectif ou dans le boitier, selon les modes de réalisation de l'invention.

La caméra 920 et l'éclairage 930 sont dirigés vers une partie du corps du patient, ici un membre 940 du patient, par exemple un bras du patient, représenté ici par un cylindre de révolution. Le bras du patient est disposé sur un support 950.

La figure 10 représente schématiquement une machine d'insertion selon un mode de réalisation de l'invention. La machine d'insertion comprend un ensemble mécatronique tel qu'un bras robotisé 16, une unité de commande 20 du bras robotisé, un système 900 de détermination selon le mode de réalisation de la figure 9, et une tête d'insertion 12. Une fois le segment d'insertion optimale déterminé, l'unité de commande 20 transmet aux actionneurs du bras robotisé 16 et aux actionneurs de la tête 12 d'insertion, les informations de déplacement du porte-aiguille de manière à permettre l'insertion de l'aiguille 14 dans la partie du corps du patient 10 à prélever.

## Revendications

1. Procédé de détermination d'au moins un segment (810a, 810b, 810c, 810d) d'insertion optimale dans un vaisseau sanguin d'un patient pour l'insertion d'une aiguille dans ledit vaisseau sanguin, ledit segment (810a, 810b, 810c, 810d) étant représentatif d'un point (820a, 820b, 820c, 820d) d'insertion dans une partie du corps du patient, d'une direction d'insertion et d'une longueur maximale d'insertion, comprenant les étapes suivantes :
- une étape (110) d'éclairage de la partie du corps du patient avec un éclairage proche infrarouge,
- une étape (120) d'acquisition d'images proche infrarouge de la partie du corps du patient avec au moins une caméra (920),
- une étape (130) de prétraitement des images acquises pour obtenir une image des vaisseaux sanguins visibles en surface de la partie du corps du patient, dite image prétraitée,
- une étape (140) d'application d'un filtre de détection de structures linéaires à ladite image prétraitée pour obtenir une image, dite carte de profil vasculaire, qui identifie les vaisseaux sanguins visibles en surface de la partie du corps du patient,
- une étape (150) de binarisation de la carte de profil vasculaire,
- une étape (170) de squelettisation des vaisseaux sanguins sur la carte de profil vasculaire binarisée, configurée pour obtenir pour chaque vaisseau sanguin un squelette dudit vaisseau sanguin,
- une étape (180) de définition de segments d'insertion à partir desdits squelettes des vaisseaux sanguins, pour chaque vaisseau sanguin,
- une étape (190) de classement des segments d'insertion en fonction de paramètres de classement prédéterminés, de sorte à identifier un ou plusieurs segments d'insertion optimale.

2. Procédé de détermination selon la revendication 1, **caractérisé en ce que** les paramètres de classements prédéterminés pour le classement des segments (810a, 810b, 810c, 810d) d'insertion sont choisis parmi un ou plusieurs paramètres de la liste suivante :
- la localisation du segment par rapport à un schéma connu de positions de vaisseaux sanguins sur la partie du corps du patient ;
- l'intensité moyenne de l'ensemble des points du vaisseau sanguin compris à l'intérieurs de contours du vaisseau sanguin correspondant au segment, calculée sur la carte de profil vasculaire ;
- la longueur du segment ;
- la profondeur du vaisseau sanguin au niveau du segment ;
- le diamètre du vaisseau sanguin au niveau du segment ;
- l'orientation du segment ;
- la présence ou l'absence d'aspérités de la peau sur le segment d'insertion ;
- une préférence du patient ;
- un historique d'insertion précédente au même patient.

3. Procédé de détermination selon l'une des revendications 1 ou 2, **caractérisé en ce que** le filtre de détection de structures linéaires est un filtre de Frangi.

4. Procédé de détermination selon l'une des revendications 1 à 3, **caractérisé en ce que** l'étape (180) de définition de segments d'insertion à partir des squelettes des vaisseaux sanguins comprend :
- une sous-étape de création d'un nœud pour chaque point de chaque squelette ;
- une sous-étape de caractérisation de chaque nœud pour former un graphe, un nœud étant un point terminal s'il n'est relié qu'à un seul nœud, une branche étant formée d'un ensemble de nœuds reliés entre eux n'ayant que deux nœuds voisins, chaque branche étant pondérée par le nombre de nœuds qui la forme ;
- une sous-étape de vérification de chaque graphe par comparaison de chaque branche avec le vaisseau sanguin correspondant sur l'image binarisée ;
- une sous-étape de correction de chaque branche non-centrée sur le vaisseau sanguin correspondant par division de la branche en nouvelles branches et création de nœuds de jonction entre les chaque nouvelles branches ;
- une sous-étape de définition des segments, un segment correspondant à une branche centrée sur son vaisseau sanguin correspondant et de longueur supérieure à un paramètre prédéterminé.

5. Procédé de détermination selon l'une des revendications 1 à 4, **caractérisé en ce que** la caméra (920) est monochromatique et équipée d'un filtre passe-haut proche infrarouge.

6. Système de détermination d'au moins un segment d'insertion optimale dans un vaisseau sanguin d'un patient pour l'insertion d'une aiguille dans ledit vaisseau, ledit segment étant représentatif d'un point d'insertion dans une partie du corps du patient, d'une direction d'insertion et d'une longueur maximale d'insertion, comprenant une unité d'acquisition d'images de la partie du corps du patient et une unité de traitement des images acquises par ladite unité d'acquisition d'images,
**caractérisé en ce que** ladite unité d'acquisition d'images comprend :
- un éclairage (930) proche infrarouge configuré pour éclairer la partie du corps du patient avec un éclairage proche infrarouge, et
- au moins une caméra (920) configurée pour acquérir des images proche infrarouges de la partie du corps du patient,
et **en ce que** l'unité (910) de traitement d'image comprend :
- un module de prétraitement d'images configuré pour pouvoir fournir une image des vaisseaux sanguins visibles en surface de la partie du corps du patient, dite image prétraitée,
- un module de filtrage, configuré pour appliquer un filtre de détection de structures linéaires à ladite image prétraitée pour obtenir une image, dite carte de profil vasculaire, qui identifie les vaisseaux sanguins visibles en surface de la partie du corps du patient,
- un module de binarisation de la carte de profil vasculaire,
- un module de squelettisation des vaisseaux sanguins sur la carte de profil vasculaire binarisée, pour obtenir pour chaque vaisseau sanguin un squelette dudit vaisseau sanguin,
- un module de définition de segments d'insertion à partir desdits squelettes des vaisseaux sanguins, pour chaque vaisseau sanguin, et
- un module de classement des segments d'insertion en fonction de paramètres de classement prédéterminés, configuré pour identifier un ou plusieurs segments d'insertion optimale.

7. Système de détermination selon la revendication 6, **caractérisé en ce que** la caméra (920) est monochromatique et équipée d'un filtre passe-haut proche infrarouge.

8. Machine d'insertion automatique ou semi-automatique, pour l'insertion d'une aiguille dans une partie du corps d'un patient (10), comprenant un ensemble (16) mécatronique, une unité (20) de commande dudit ensemble (16) mécatronique, et une tête (12) d'insertion d'une aiguille montée sur l'ensemble (16) mécatronique, **caractérisé en ce qu'**elle comprend en outre, un système (900) de détermination selon l'une des revendications 6 ou 7, configuré pour déterminer un segment d'insertion optimale de l'aiguille dans la partie du corps du patient (10).

## Patentansprüche

1. Verfahren zum Bestimmen mindestens eines optimalen Einführungsabschnitts (810a, 810b, 810c, 810d) in einem Blutgefäß eines Patienten zum Einführen einer Nadel in das Blutgefäß, wobei der Abschnitt (810a, 810b, 810c, 810d) repräsentativ für einen Einführungspunkt (820a, 820b, 820c, 820d) in einem Teil des Körpers des Patienten, eine Einführungsrichtung und eine maximale Einführungslänge ist, wobei das Verfahren die Schritte umfasst:
- einen Schritt (110) des Beleuchtens des Körperteils des Patienten mit Nahinfrarot-Beleuchtung,
- einen Schritt (120) des Erfassens von Nahinfrarotbildern des Körperteils des Patienten mit mindestens einer Kamera (920),
- einen Schritt (130) der Vorverarbeitung der aufgenommenen Bilder, um ein Bild der auf der Oberfläche des Körperteils des Patienten sichtbaren Blutgefäße zu erhalten, das als vorverarbeitetes Bild bezeichnet wird,
- einen Schritt (140) der Anwendung eines linearen Strukturerkennungsfilters auf das vorverarbeitete Bild, um ein Bild zu erhalten, das als Gefäßprofilkarte bezeichnet wird, der auf der Oberfläche des Körperteils des Patienten sichtbare Blutgefäße identifiziert,
- einen Schritt (150) der Binärisierung der Gefäßprofilkarte,
- einen Schritt (170) der Skelettierung der Blutgefäße auf der binärisierten Gefäßprofilkarte, der ausgestaltet ist, so dass für jedes Blutgefäß ein Skelett des Blutgefäßes erhalten wird,
- einen Schritt (180) zum Definieren von Einführungsabschnitten aus den Skeletten der Blutgefäße für jedes Blutgefäß,
- einen Schritt (190) des Klassifizierens der Einführungsabschnitte gemäß bestimmter Klassifizierungsparameter, um so ein oder mehrere optimale Einführungsabschnitte zu identifizieren.

2. Bestimmungsverfahren nach Anspruch 1, **dadurch gekennzeichnet, dass** die bestimmten Klassifizierungsparameter zum Klassifizieren der Einführungsabschnitte (810a, 810b, 810c, 810d) aus einem oder mehreren Parametern aus der folgenden Liste ausgewählt werden:
- der Lage des Abschnitts in Bezug auf ein bekanntes Muster von Positionen von Blutgefäßen auf dem Körperteil des Patienten;
- der durchschnittlichen Dichte aller Punkte des Blutgefäßes, die in den Konturen des Blutgefäßes enthalten sind, das dem Abschnitt entspricht, berechnet auf der Gefäßprofilkarte,
- der Länge des Abschnitts;
- der Tiefe des Blutgefäßes in dem Abschnitt;
- dem Durchmesser des Blutgefäßes in dem Abschnitt;
- der Ausrichtung des Abschnitts;
- dem Vorhandensein oder Nichtvorhandensein von Unregelmäßigkeiten auf der Haut auf dem Einführungsabschnitt.
- einer Präferenz des Patienten;
- einer früheren Einführungshistorie bei demselben Patienten.

3. Bestimmungsverfahren nach einem der Ansprüche 1 oder 2, **dadurch gekennzeichnet, dass** der lineare Strukturerkennungsfilter ein Frangi-Filter ist.

4. Bestimmungsverfahren nach einem der Ansprüche 1 bis 3, **dadurch gekennzeichnet, dass** der Schritt (180) des Definierens von Einführungsabschnitten aus den Skeletten der Blutgefäße umfasst:
- einen Teilschritt des Erzeugens eines Knotens für jeden Punkt eines jeden Skeletts;
- einen Teilschritt der Charakterisierung jedes Knotens, um einen Graphen zu bilden, worin ein Knoten ein Endpunkt ist, wenn er nur mit einem einzigen Knoten verbunden ist, worin ein Zweig aus einer Menge von miteinander verbundenen Knoten gebildet ist, die nur zwei benachbarte Knoten haben, worin jeder Zweig durch die Anzahl von Knoten gewichtet wird, die ihn bilden;
- einen Teilschritt der Verifizierung jedes Graphen durch Vergleich jedes Zweiges mit dem entsprechenden Blutgefäß auf dem binärisierten Bild;
- einen Teilschritt zum Korrigieren jeder nicht zentrierten Verzweigung auf dem entsprechenden Blutgefäß durch Aufteilen der Verzweigung in neue Verzweigungen und Erzeugen von Verbindungsknoten zwischen jeder der neuen Verzweigungen;
- einen Teilschritt zum Definieren von Abschnitten, worin ein Abschnitt einem Zweig entspricht, der auf seinem entsprechenden Blutgefäß zentriert ist und eine Länge aufweist, die größer als ein bestimmter Parameter ist.

5. Bestimmungsverfahren nach einem der Ansprüche 1 bis 4, **dadurch gekennzeichnet, dass** die Kamera (920) monochromatisch und mit einem Nahinfrarot-Hochpassfilter ausgestattet ist.

6. System zur Bestimmung mindestens eines optimalen Einführungsabschnitts in ein Blutgefäß eines Patienten zum Einführen einer Nadel in das Gefäß, wobei der Abschnitt für einen Einführungspunkt in einem Körperteil des Patienten, eine Einführungsrichtung und eine maximale Einführungslänge repräsentativ ist, welches eine Einheit zur Erfassung von Bildern des Körperteils des Patienten und eine Einheit zur Verarbeitung der von der Bilderfassungseinheit erfassten Bilder umfasst,
**dadurch gekennzeichnet, dass** die Bilderfassungseinheit umfasst:
- eine Nahinfrarot-Beleuchtung (930), die ausgestaltet ist, den Teil des Körpers des Patienten mit Nahinfrarot-Beleuchtung zu beleuchten, und
- mindestens eine Kamera (920), die ausgestaltet ist, Nahinfrarot-Bilder des Körperteils des Patienten aufzunehmen,
und darin, dass die Bildverarbeitungseinheit (910) umfasst:
- ein Modul zur Vorverarbeitung von Bildern, das ausgestaltet ist, ein Bild der auf der Oberfläche des Körperteils des Patienten sichtbaren Blutgefäße zu liefern, das als vorverarbeitetes Bild bezeichnet wird,
- ein Modul zur Filterung, das ausgestaltet ist, einen linearen Strukturerkennungsfilter auf das vorverarbeitete Bild anzuwenden, um ein Bild zu erhalten, das als Gefäßprofilkarte bezeichnet wird und die Blutgefäße identifiziert, die auf der Oberfläche des Körperteils des Patienten sichtbar sind,
- ein Modul zur Binärisierung der Gefäßprofilkarte,
- ein Modul zur Skelettierung der Blutgefäße auf der binärisierten Gefäßprofilkarte, um für jedes Blutgefäß ein Skelett des Blutgefäßes zu erhalten,
- ein Modul zum Definieren von Einführungsabschnitten aus den Skeletten der Blutgefäße für jedes Blutgefäß, und
- ein Modul zum Klassifizieren der Einführungsabschnitte gemäß bestimmter Klassifizierungsparameter, das ausgestaltet ist, ein oder mehrere optimale Einführungsabschnitte zu identifizieren.

7. Bestimmungssystem nach Anspruch 6, **dadurch gekennzeichnet, dass** die Kamera (920) monochromatisch und mit einem Nahinfrarot-Hochpassfilter ausgestattet ist.

8. Automatische oder halbautomatische Einführungsmaschine zum Einführen einer Nadel in einen Körperteil eines Patienten (10), welche eine mechatronische Baugruppe (16), eine Einheit (20) zum Steuern der mechatronischen Baugruppe (16) und einen Einführungskopf (12) für eine Nadel umfasst, der an der mechatronischen Baugruppe (16) angebracht ist, **dadurch gekennzeichnet, dass** diese ferner ein Bestimmungssystem (900) nach einem der Ansprüche 6 oder 7 umfasst, das ausgestaltet ist, einen optimalen Einführungsabschnitt zum Einführen der Nadel in den Körperteil des Patienten (10) zu bestimmen.

## Claims

1. Method for determining at least one optimal insertion segment (810a, 810b, 810c, 810d) in a blood vessel of a patient for inserting a needle into said blood vessel, said segment (810a, 810b, 810c, 810d) being representative of an insertion point (820a, 820b, 820c, 820d) in a part of the body of the patient, an insertion direction and a maximum insertion length, comprising the following steps:
- a step (110) of illuminating the part of the body of the patient with near-infrared illumination,
- a step (120) of acquiring near-infrared images of the part of the body of the patient with at least one camera (920),
- a step (130) of pre-processing the acquired images to obtain an image of the blood vessels visible on the surface of the part of the body of the patient, referred to as pre-processed image,
- a step (140) of applying a linear structure detection filter to said pre-processed image to obtain an image, referred to as vascular profile map, which identifies the blood vessels visible on the surface of the part of the body of the patient,
- a step (150) of binarizing the vascular profile map,
- a step (170) of skeletonising the blood vessels on the binarized vascular profile map, configured to obtain, for each blood vessel, a skeleton of said blood vessel,
- a step (180) of defining insertion segments from said skeletons of the blood vessels, for each blood vessel,
- a step (190) of classifying the insertion segments according to predetermined classification parameters, so as to identify one or more optimal insertion segments.

2. Determining method as claimed in claim 1, **characterised in that** the predetermined classification parameters for classifying the insertion segments (810a, 810b, 810c, 810d) are selected from one or more parameters from the following list:
- the location of the segment with respect to a known pattern of positions of blood vessels on the part of the body of the patient;
- the average density of all of the points of the blood vessel included within contours of the blood vessel corresponding to the segment, calculated on the vascular profile map;
- the length of the segment;
- the depth of the blood vessel in the segment;
- the diameter of the blood vessel in the segment;
- the orientation of the segment;
- the presence or absence of irregularities on the skin on the insertion segment.
- a preference of the patient;
- a previous insertion history for the same patient.

3. Determining method as claimed in any one of claims 1 or 2, **characterised in that** the linear structure detection filter is a Frangi filter.

4. Determining method as claimed in any one of claims 1 to 3, **characterised in that** the step (180) of defining insertion segments from the skeletons of the blood vessels comprises:
- a sub-step of creating a node for each point of each skeleton;
- a sub-step of characterising each node to form a graph, a node being a terminal point if it is connected to only a single node, a branch being formed from a set of nodes connected together having only two neighbouring nodes, each branch being weighted by the number of nodes which form it;
- a sub-step of verifying each graph by comparing each branch with the corresponding blood vessel on the binarized image;
- a sub-step of correcting each non-centred branch on the corresponding blood vessel by dividing the branch into new branches and creating junction nodes between each of the new branches;
- a sub-step of defining segments, one segment corresponding to a branch centred on its corresponding blood vessel and having a length greater than a predetermined parameter.

5. Determining method as claimed in any one of claims 1 to 4, **characterised in that** the camera (920) is monochromatic and equipped with a near-infrared high-pass filter.

6. System for determining at least one optimal insertion segment in a blood vessel of a patient for inserting a needle into said vessel, said segment being representative of an insertion point in a part of the body of the patient, an insertion direction and a maximum insertion length, comprising a unit for acquiring images of the part of the body of the patient and a unit for processing the images acquired by said image acquiring unit,
**characterised in that** said image acquiring unit comprises:
- near-infrared illumination (930) configured to illuminate the part of the body of the patient with near-infrared illumination, and
- at least one camera (920) configured to acquire near-infrared images of the part of the body of the patient,
and **in that** the image processing unit (910) comprises:
- a module for pre-processing images configured to be able to provide an image of the blood vessels visible on the surface of the part of the body of the patient, referred to as pre-processed image,
- a module for filtering, configured to apply a linear structure detection filter to said pre-processed image to obtain an image, referred to as vascular profile map, which identifies the blood vessels visible on the surface of the part of the body of the patient,
- a module for binarizing the vascular profile map,
- a module for skeletonising the blood vessels on the binarized vascular profile map, in order to obtain, for each blood vessel, a skeleton of said blood vessel,
- a module for defining insertion segments from said skeletons of the blood vessels, for each blood vessel, and
- a module for classifying the insertion segments according to predetermined classification parameters, configured to identify one or more optimal insertion segments.

7. Determining system as claimed in claim 6, **characterised in that** the camera (920) is monochromatic and equipped with a near-infrared high-pass filter.

8. Automatic or semi-automatic insertion machine for the insertion of a needle into a part of the body of a patient (10), comprising a mechatronic assembly (16), a unit (20) for controlling said mechatronic assembly (16), and an insertion head (12) for a needle mounted on the mechatronic assembly (16), **characterised in that** it further comprises a determining system (900) as claimed in any one of claims 6 or 7, configured to determine an optimal insertion segment for inserting the needle into the part of the body of the patient (10).
